Europäisches Patentamt

European Patent Office

Office européen des brevets

(11) Publication number: **0 372 972 B1**

(12) **EUROPEAN PATENT SPECIFICATION**

(45) Date of publication of patent specification: **02.02.94**

(51) Int. Cl.5: **C07C 255/08**, C07C 253/26, C07C 253/34, C07D 303/04, C07D 301/08

(21) Application number: **89312787.8**

(22) Date of filing: **07.12.89**

(54) **Process for the production of nitriles and oxides.**

(30) Priority: **08.12.88 US 281581**
**21.09.89 US 410435**

(73) Proprietor: **THE BOC GROUP, INC.**
**575 Mountain Avenue**
**Murray Hill New Jersey 07974(US)**

(43) Date of publication of application:
**13.06.90 Bulletin 90/24**

(72) Inventor: **Ramachandran, Ramakrishnan**
**232 Hillside Avenue**
**Allendale New Jersey, 07401(US)**
Inventor: **Malik, Virginia A.**
**1633 Lenape Road**
**Linden New Jersey 07036(US)**
Inventor: **MacLean, Donald L.**
**102 Petticoat Lane**
**Annandale New Jersey 08801(US)**
Inventor: **Satchell, Donald P., Jr.**
**10 Lavina Court**
**Summit New Jersey 07901(US)**

(45) Publication of the grant of the patent:
**02.02.94 Bulletin 94/05**

(84) Designated Contracting States:
**AT BE DE ES FR GB IT NL SE**

(56) References cited:
**EP-A- 0 318 205**
**EP-A- 0 328 280**
**EP-A- 0 336 592**
**GB-A- 1 377 735**
**US-A- 4 609 502**

(74) Representative: **Wickham, Michael et al**
**c/o Patent and Trademark Department**
**The BOC Group plc**
**Chertsey Road**
**Windlesham Surrey GU20 6HJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

The present invention is directed to an improvement in a process for producing nitriles and oxides from alkanes, an oxygen-containing gas and, where necessary, ammonia in the presence of a suitable catalyst under conditions which achieve high efficiency and selectivity toward the desired product.

The production of nitriles and oxides by ammoxidation and oxidation, respectively, of an appropriate alkene in the presence of a suitable catalyst is well known. The production of acrylonitrile, for example, from a gaseous feed of propylene, ammonia and air is described by Bruce E. Gates et al in Chemistry of Catalytic Processes, McGraw Hill (1979), pp. 380-384.

The feed is sent to an ammoxidation reactor where, in the presence of a suitable catalyst, acrylonitrile is produced along with lesser amounts of other nitrogen-containing compounds. The effluent from the ammoxidation reaction is quenched with water and the desired products are obtained in the liquid phase. The gas phase by-products, typically oxygen, carbon dioxide, carbon monoxide, nitrogen and unreacted hydrocarbon, are combined with natural gas and sent to a boiler for combustion as disclosed, for example, in US-A-3,591,620 and US-A-4,335,056. A typical ammoxidation process is illustrated in Figure 1 of the drawings.

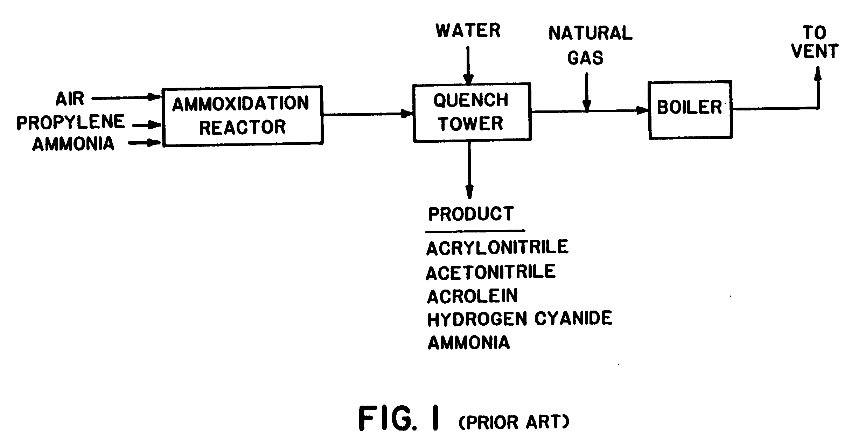

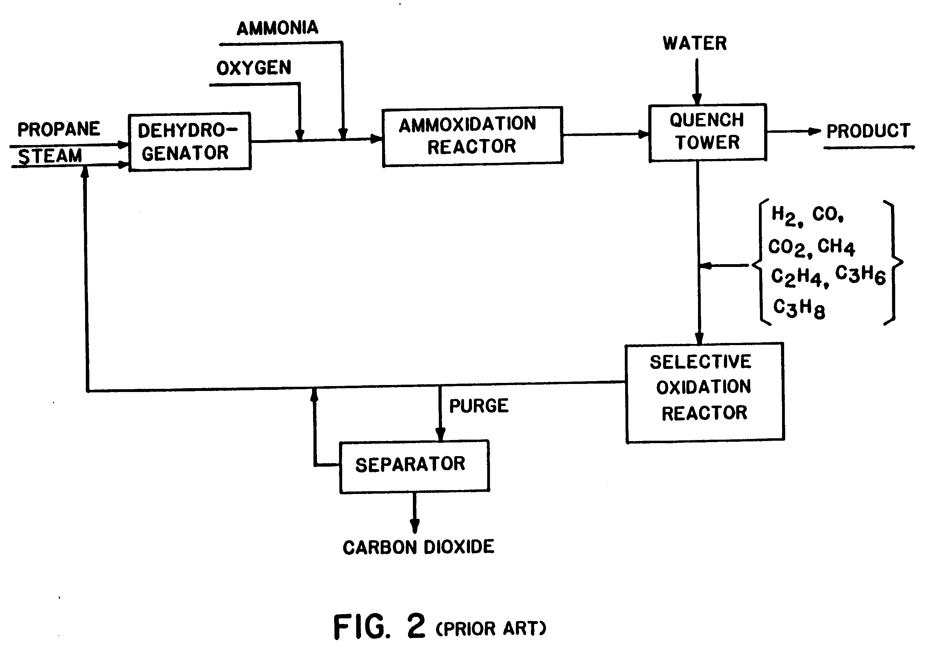

More recently, US-A-4,609,502 (hereinafter referred to as Khoobiar et al) disclosed a cyclic process for producing acrylonitrile using propane as a starting material which is initially dehydrogenated catalytically in the presence of steam to form propylene. (For ease of reference a process according to Khoobiar et al is shown in Figure 2 of the accompanying drawings.) The use of steam is in contrast to most conventional dehydrogenation processes and which avoid steam primarily due to the costs involved. After ammoxidation, the effluent is quenched, the desired product removed, and the off-gases, including propylene and propane, are sent to an oxidation reactor to remove oxygen by selective reaction with hydrogen to form water vapour. The gas mixture exiting the selective oxidation reactor includes substantial amounts of methane, ethane and ethylene, which are by-products of dehydrogenation, and unreacted propylene in addition to carbon oxides. As an option, this gas mixture is split and a portion is sent to a separator which removes only carbon dioxide. A portion of the effluent from the separator is purged to remove light hydrocarbons. The non-purged stream is mixed with the remainder of the oxidation reactor effluent, fresh propane and steam, and, if necessary, is sent to the dehydrogenator where the propane is converted to propylene. Another option is to cool and liquefy the $C_3$ hydrocarbons therefrom and then vaporise them prior to recycle.

The aforementioned process suffers from several disadvantages. For example, there is no practical way selectively to remove by-products of propane dehydrogenation, such as methane, ethane, ethylene and the like, thereby preventing their accumulation in the system other than by removing them in the purge stream. The removal of these gases in a purge stream will result in a loss of some of the circulating propane and propylene. As the process is being carried on in a continuous manner, this loss of starting material causes a significant decrease in the yield of propylene. As mentioned above, propane and propylene can be recovered from the purge stream prior to venting. This requires additional refrigeration apparatus to cool and liquefy propylene and propane. The separated $C_3$ hydrocarbons must be vaporised prior to recycle. These operations add to the capital costs and power requirements of the process.

Another disadvantage of the Khoobiar et al process stems from the use of the selective oxidation reactor to treat the gaseous effluent from the quencher. The gases exiting the quencher are at ambient temperature and must be heated prior to introduction into the oxidation reactor in order to promote oxygen removal. Because there is a significant amount of oxygen in the quench effluent, the heat of reaction generated in the oxidation reactor can result in excessive temperatures in the system. There are three options to alleviate this problem. First, the amount of oxygen entering the oxidation reactor can be reduced by other means. Second, multiple reactors can be utilised with a cooling means between each pair of reactors. Third, a portion of the effluent from the reactor is passed through a cooling means and recycled to the feed to reduce the internal temperature of the reactor. None of these measures is attractive from the viewpoint of cost and efficiency.

The oxidation reactor in the Khoobiar et al process is operated with oxidation catalysts such as noble metals (e.g., platinum). Olefins and carbon monoxide, which are generated in the dehydrogenation reactor, are known to deactivate these catalysts, as disclosed in Catalytic Processes and Proven Catalysts, Charles L. Thomas, Academic Press (1970) pp. 118-119. Accordingly, multiple oxidation reactors must be used to allow for frequent regeneration of the catalyst which represents yet another addition to production costs (US. Patent No. 4,609,502, column 4, lines 51-56).

It is therefore apparent that the industry is still searching for a cost effective process of converting hydrocarbons into nitriles or oxides. Applicants have discovered an improvement in processes which are cost effective and in which the disadvantages of the aforementioned systems are substantially reduced or

EP 0 372 972 B1

eliminated. Moreover, in comparison to conventional processes, the thermal requirements of the improved processes of the invention are markedly reduced.

The processes improved in accordance with the invention provide for the production of nitriles and oxides by converting a gaseous alkane to the corresponding alkene in a dehydrogenator, reacting the alkene in an ammoxidation/oxidation reactor with an oxygen-containing gas, preferably oxygen-enriched air, and if nitriles are to be produced, ammonia gas in the presence of a suitable catalyst to form the desired product. The product stream is quenched with a liquid to form a liquid phase containing the desired product and a gas phase which is passed under pressure into a separator which provides an oxygen-containing stream, a product stream containing reactant alkane and alkene hydrocarbons, and a vent stream containing carbon oxides and lower hydrocarbons. A hydrogen-enriched stream may also be produced, if desired. Nitrogen, when present in the feed stream, is removed in the oxygen-containing stream. The product stream is passed into a selective oxidation unit where minor amounts of residual oxygen are removed. The effluent from the selective oxidation unit is recycled to the dehydrogenator with fresh propane feed. The oxygen-containing stream from the separator may be recycled to the oxidation/ammoxidation reactor, depending on the nitrogen content thereof. In one embodiment of the present invention, the separator system produces a hydrogen-enriched stream which may be recycled to the dehydrogenator, the selective oxidation unit or withdrawn as product. The dehydrogenator may be a single unit or a multistage unit wherein the recycle stream is admitted to a particular stage following that from which the effluent for the reactor is withdrawn. A second separator system may be present between the dehydrogenator and the reactor to remove hydrogen from the dehydrogenator effluent. When the second separator system is present, the first separator system does not produce a hydrogen-enriched stream. Each separator may be one or more separation units known to those skilled in the art, e.g., an absorber, a cryogenic distillation column, a membrane separator or a pressure swing adsorber (PSA). Note that, while streams are designated with respect to certain components of interest, the streams will contain others in at least trace amounts which do not affect operation of the process of the present invention. For example, carbon dioxide will be present in the recycle hydrocarbon stream.

According to the invention there is provided a process for the production of a nitrile or an oxide comprising the steps of:

a) catalytically dehydrogenating gaseous alkane so as to form a gas mixture comprising alkene and unreacted alkane;

b) reacting in the presence of a suitable catalyst the alkene content of the gas mixture with oxygen and, if a nitrile is to be produced, ammonia so as to form a gaseous reaction effluent containing said oxide or said nitrile, the source of the oxygen being pure oxygen, air or a gas enriched in oxygen relative to air;

c) quenching said effluent in a liquid to form a liquid phase containing said nitrile or oxide and a gaseous phase;

d) recovering said nitrile or oxide from said liquid phase;

e) separating the gaseous phase into a first stream comprising said alkane, said alkene, and, as an impurity, oxygen; a second stream comprising 'waste' gas; and a third stream comprising oxygen;

f) either (i) introducing the first stream into a catalytic selective oxidation unit to remove the remaining oxygen in said stream; and recycling the effluent from the selective oxidation unit to the dehydrogenator, or (ii) passing the first stream directly to the dehydrogenator.

The process of this invention is applicable to the synthesis of nitriles and oxides. In each instance, an alkene is reacted with an oxygen-containing gas comprising pure oxygen, air or a gas enriched in oxygen (relative to air) in the presence of a suitable catalyst. The term "suitable catalyst" indicates a catalyst that will catalyse the production of the desired product under the conditions utilised in the reactor. In the event an oxide, e.g. ethylene oxide, is desired, an oxidation catalyst is utilised. On the other hand, to form a nitrile, the feed to the reactor additionally includes ammonia and the catalyst is an ammoxidation catalyst. These catalysts and their use are conventional and well known to one of ordinary skill in the art.

Illustrative of products, and their respective starting gaseous alkanes, which can be advantageously produced by the method of this invention are acrylonitrile from propane, methacrylonitrile from isobutane, ethylene oxide from ethane.

The invention will now be described by way of example with reference to the accompanying drawings in which:

FIG. 3 illustrates in a block diagram the improvement of the invention in a process for producing acrylonitrile wherein a selective oxidation unit is downstream of the PSA unit.

FIG. 4 illustrates in a block diagram the process of FIG. 3 wherein an additional PSA unit removes hydrogen from the dehydrogenator effluent.

3

FIG. 5 illustrates in a block diagram the improvement of the invention in a process for producing acrylonitrile utilising a multistage dehydrogenator without a selective oxidation unit downstream of the PSA unit.

FIG. 6 illustrates in a block diagram detail of the multistage dehydrogenator shown in Fig. 5.

In the interest of brevity, the subject process will be described with reference to the production of acrylonitrile from propane, but is in no way intended to be limited thereto.

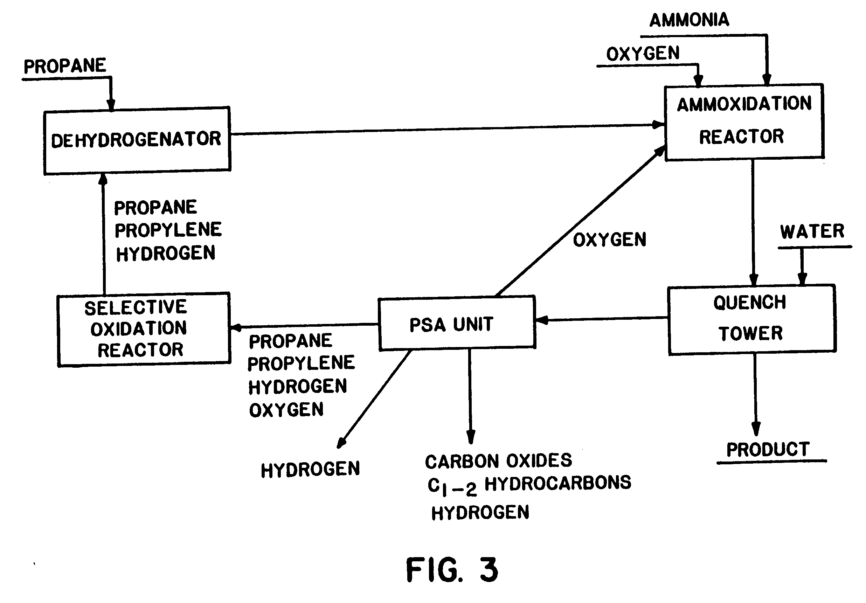

The process shown in FIG. 3 provides the efficiency of recycle afforded by the process illustrated in FIG. 2 and is similar in a generic sense in that it contains the same kinds of functional units, yet is substantially more efficient and, unexpectedly, capable of effectively utilising air or oxygen-enriched air as a feed to the ammoxidation/oxidation reactor. Specifically, one embodiment of the subject process utilises a pressure swing adsorption (PSA) system having an operating cycle such that it will produce an oxygen-containing stream ('the third stream') which will also contain nitrogen, if present in the feed stream, a product stream ('the first stream') containing unreacted alkane/alkene hydrocarbons from the gaseous quench tower effluent, a vent stream containing carbon oxides and lower hydrocarbons ('the second stream') and a hydrogen-enriched stream ('the fourth stream'). These streams can be recycled to the oxidation/ammoxidation reactor, the dehydrogenator or the selective oxidation unit, as appropriate. Since the product stream contains only a small quantity, i.e. typically 1-2 percent by volume, of oxygen, the selective oxidation reactor can be comparatively small in terms of capital expenditure and have a long life, yet still function effectively. The configuration of the subject process eliminates the substantial loss of efficiency inherent in the process of FIG. 2 by the use of the purge stream. In addition, the novel operation of the PSA or other separation system of the subject invention provides for recycle of an oxygen-enriched stream ("the third stream"), thus providing a further increase in process efficiency. The hydrogen-enriched stream may be recycled as well or withdrawn as product.

Referring to FIG. 3, propane is fed into the dehydrogenator where it is converted to propylene. For increased catalyst life, a hydrogen-containing gas may be introduced into the dehydrogenator with the propane feed. The required amount of hydrogen can conveniently be provided through the recycle stream from the PSA system as will be discussed below. The hydrogen-containing gas can, if desired, be provided as a discrete stream. The dehydrogenator can be of any design including a multistage dehydrogenator, as will be discussed hereafter. The catalyst utilised in the dehydrogenator can be any conventional dehydrogenation catalyst, preferably one or more Group VIII noble metals such as platinum on an alumina support. A steam-assisted dehydrogenator may be utilised as well.

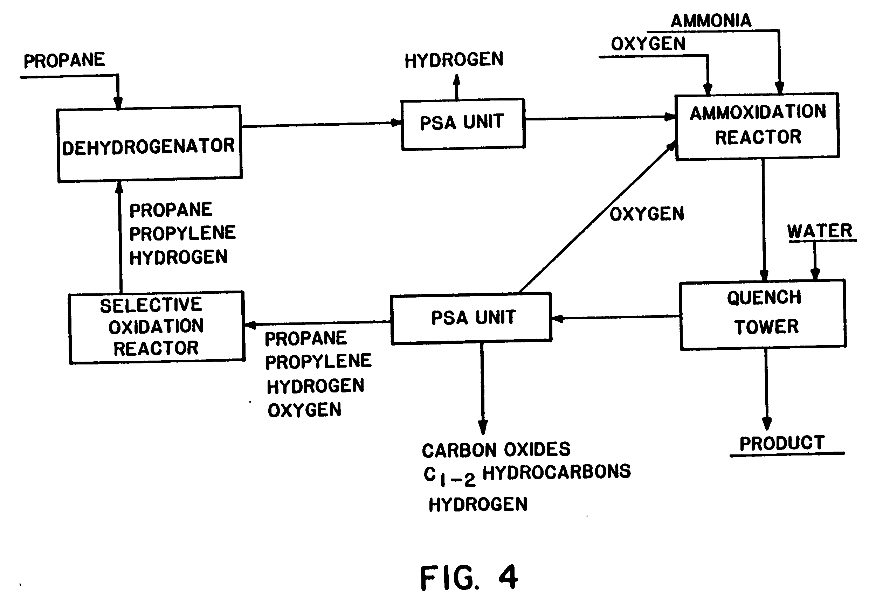

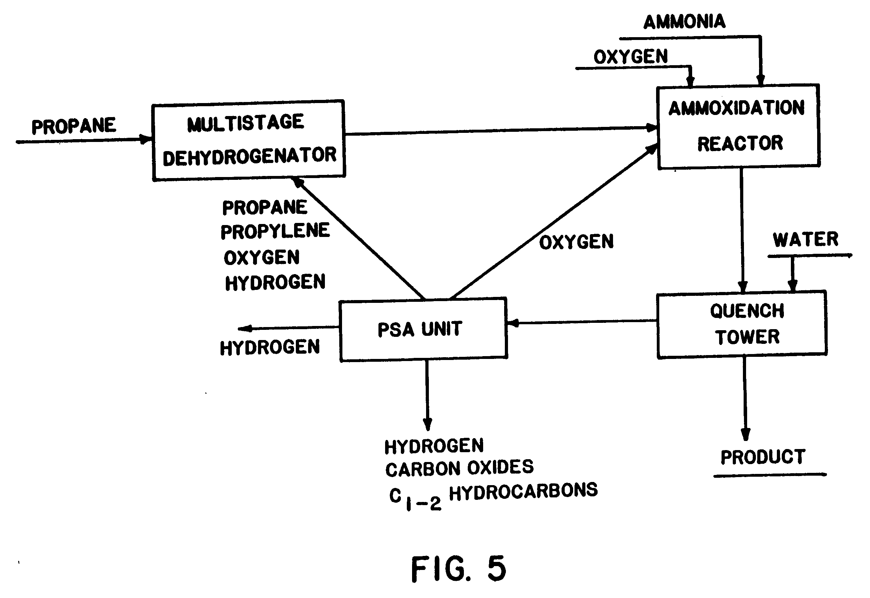

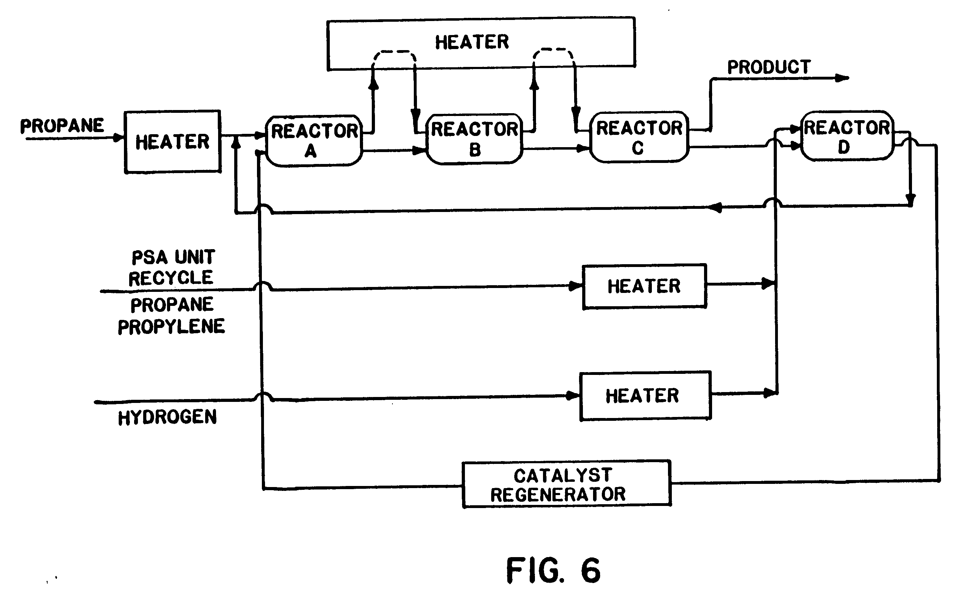

The effluent product stream from the dehydrogenator, comprising unreacted propane, propylene and hydrogen, is fed into a conventional ammoxidation/oxidation reactor along with (a) pure oxygen, air or, preferably, oxygen-enriched air and (b) ammonia. The system shown in FIGS. 3 and 4 utilises pure oxygen as a feed. In the event that an oxygen-enriched stream is recycled from the PSA or other separation system, it may be introduced independently or in combination with the oxygen feed. The relative proportions of each can be adjusted to achieve the desired amount of oxygen in the reactor. In the event that the feed to the oxidation/ammoxidation reactor is air or oxygen-enriched air, the oxygen-containing stream produced by the PSA system may be vented or only partially recycled to the reactor. The amount of the oxygen-containing stream produced by the PSA or other separation system which is vented will depend on the oxygen content of the reactor feed and the desired reactor pressure. The venting of the oxygen-containing stream through a purge line, not shown, thereby prevents the accumulation of nitrogen in the system. These considerations apply as well to the system shown in FIGS. 5 and 6 which is also illustrated with a pure oxygen feed.

The ammoxidation/oxidation reactor utilised in the present process is conventional and may employ either a fixed or fluidized or transport catalyst bed. A typical example of an ammoxidation reactor is disclosed in Angstadt et al., US. Patent No. 4,070,393 and Gates et al., ibid, pp. 381-383, each incorporated herein by reference. The reactor contains a conventional ammoxidation catalyst, such as bismuth-molybdenum oxide, iron-antimony oxide, uranium antimony oxide precipitated on silica. Other suitable catalysts are disclosed, for example, in Chemistry of Catalytic Processes, Gates et al, McGraw Hill (1979) pp 349-350, and Yoshino et al, US. Patent No. 3,591,620, incorporated herein by reference. Additional suitable catalysts are known to those skilled in the art.

The ammoxidation reaction is conducted at a temperature of from 375° to 550°C., preferably from 400° to 500°C., at low pressures, typically in the range of from 122 to 308 kPa (3 to 30 psig), preferably from 136 to 239 kPa (5 to 20 psig). The reactants are passed through the reactor at a relatively low velocity, typically in the range of from 533 to 671 mm/s (1.75 to 2.2 ft/sec). The oxygen-containing gas feed may be pure oxygen, air or oxygen-enriched air. In accordance with this invention, oxygen-enriched air preferably contains from 30 to 80, most preferably from 55 to 65, percent by volume of oxygen. Such mixtures may be produced by adjusting the capacity of a conventional oxygen-producing unit, e.g. a conventional PSA unit, or by mixing pure oxygen with air in the proper proportions. The ratio of oxygen to propylene in the feed is suitably in the range of from 1.6:1 to 2.4:1 by volume. In the production of a nitrile, the ratio of ammonia to propylene in the feed is suitably in the range of from 0.7 to 1.2:1 by volume.

The effluent from the ammoxidation reactor comprises a major amount of acrylonitrile and minor amounts of acrolein, hydrogen cyanide, acetonitrile, carbon oxides and nitrogen, when present in the feed, as well as unreacted oxygen, propylene and propane. This gaseous mixture is quenched or scrubbed with a liquid, such as water, to dissolve the water-soluble compounds for subsequent separation and recovery of acrylonitrile, acetonitrile and hydrogen cyanide. The quench liquid may be made slightly acidic with a suitable acid, such as sulphuric acid, to aid in the removal of ammonia as is known in the art.

The gas phase effluent from the quench step ('the gaseous phase') is introduced into a separator, which utilises a membrane cell, absorber, a PSA apparatus or a distillation column operating at cryogenic temperatures. These separators can be used alone or in combination, depending on whether pure oxygen or enriched air is utilised as the feed as is well known to those skilled in the art. For example, when enriched air is used as the feed, membrane separation followed or preceded by a PSA separation can be used to prevent any $N_2$ accumulation. For purposes of the following illustration, a PSA system will be used. As used herein, a PSA system comprises two or more adsorbent bed units in series, each unit comprising one or more adsorbent beds in parallel.

PSA is a well known process for separating the components of a mixture of gases by virtue of the difference in the degree of adsorption among them on a particular adsorbent retained in a stationary bed. Typically, two or more such beds are operated in parallel as a cyclic process comprising adsorption under relatively high pressure and desorption or bed regeneration under low pressure or vacuum. The desired component or components may be obtained during either of these stages. The cycle may contain other steps in addition to the fundamental steps of adsorption and regeneration, and it is commonplace to have two or more adsorbent beds cycled out of phase to assure a pseudo-continuous flow of desired product. It is preferred to pass the quench tower effluent through a conventional dryer (not shown) to remove moisture therefrom prior to introducing it into the PSA.

It may be necessary to raise the pressure of the quench tower effluent in a compressor or other suitable means prior to introducing it into the PSA system. The compressor increases the pressure of the quench tower gaseous effluent to the operating pressure of a PSA system, typically from 122 kPa to 446 kPa (3 to 50 psig), preferably from 239 to 377 kPa (20 to 40 psig). These ranges may vary to an extent depending on the adsorbent in the PSA system. It may also be necessary to pass the effluent through a conventional dryer, not shown, prior to introduction into the PSA system.

The PSA system utilised in accordance with the present invention comprises at least two adsorptive beds functioning in series. Of course, these beds in series may be physically located as discrete layers within a single vessel. The quench tower effluent entering the PSA system consists of propane; propylene; hydrogen; light hydrocarbons, e.g. ethane, ethylene and methane; carbon monoxide; carbon dioxide; oxygen and, if present in the feed to the oxidation reactor, nitrogen. The adsorbent in the first bed can be any material recognised in the art which will adsorb $C_3$ hydrocarbons preferentially to the other gases, thereby enabling these to be produced by subsequent desorption a stream containing alkane, alkene, minor quantities of oxygen and, when present in the gaseous phase, nitrogen, ('the first stream'). Silica gel and activated carbon are preferred adsorbent materials. Silica gel is a particularly preferred material wherein oxygen-enriched air is utilised as a reactor feed material. A non-adsorbed stream containing hydrogen, oxygen and if present in the gaseous phase, nitrogen, flows through the first bed and is introduced into the second bed.

The PSA system is operated such that the non-adsorbed stream flowing through the first bed is not flammable. In the step of the PSA cycle, after adsorption, the pressure in the bed is lowered to a value such that a waste stream, which contains the remaining light hydrocarbons and carbon dioxide, can be withdrawn from the outlet of the bed with minimal desorption of product hydrocarbons ('the second stream'). The waste stream may be either vented or incinerated. The first stream is then produced conventionally at the PSA feed gas inlet by further pressure reduction desorbing the bed, and is utilised for recycle.

The adsorbent in the second bed of the PSA system is selected so that it will adsorb oxygen, and, if present, nitrogen in preference to hydrogen to form the third stream upon desorption. For example, a molecular sieve zeolite may be used. The second bed is operated in such a manner that the oxygen in the non-adsorbed effluent flowing therethrough is below flammability levels. The second bed produces a recycle stream of predominately oxygen and nitrogen when present, and a stream rich in hydrogen. The latter stream may be vented, taken as product or recycled to the dehydrogenator to maintain a desired level of hydrogen therein. The PSA system is operated in a manner such that none of the streams produced thereby is flammable and the concentration of oxygen and hydrogen in their respective streams is maximised. For example, flammability of the oxygen-containing stream is suppressed by withdrawing it fuel-rich, i.e. containing a high percentage of hydrogen and light hydrocarbons.

Since the hydrogen-rich stream produced in the PSA system may contain some oxygen, it is not introduced directly into the dehydrogenator, but is instead introduced into the selective oxidation reactor with the hydrocarbon recycle stream. The amount of hydrogen required in the recycle feed to the dehydrogenator or the selective oxidation reactor will vary with the catalyst and can be determined by operation of the system utilising a given catalyst.

In the event that the feed to the ammoxidation/ oxidation reactor is pure oxygen, the PSA system produces an oxygen-enriched stream of product quality which is recycled to the reactor. As the percentage of nitrogen in the reactor feed increases, however, the amount of the oxygen stream that is recycled decreases to prevent the accumulation of nitrogen in the system. Even in the event that the oxygen-containing stream produced in the PSA system is totally purged, the process of the invention is advantageous in that oxygen loss is minimal and nitrogen build-up is prevented without the loss of an appreciable amount of reactant hydrocarbons.

While it is preferred that the two adsorbent beds in the PSA system be contained in separate vessels, it is within the scope of the present invention to utilise two discrete layers of adsorbent in a single vessel configured to provide the streams as described herein. In such a two layered vessel, for example, the waste stream containing light hydrocarbons and carbon oxides would be withdrawn from an intermediate level of the vessel without passing through the second layer.

The PSA product stream ('the first stream') withdrawn from the first bed of the PSA system and introduced into the selective oxidation reactor contains propane, propylene, a minor quantity of oxygen, typically about 1-2 percent by volume, and, if present in the feed, nitrogen. The selective oxidation reactor is of conventional configuration and contains a catalyst known in the art to be capable of selectively catalysing the reaction of oxygen and hydrogen to form water, i.e. the oxidation of hydrogen, without causing oxidation of the desired hydrocarbons, i.e. propane and propylene in the PSA effluent. Such catalysts and their use are well known. Suitable catalysts include noble metals or base metals, particularly platinum or palladinm on alumina.

As previously stated, the selective oxidation reactor utilised in the embodiment of the present process shown in Fig. 3 requires only a modest capital expenditure in comparison with the multiple bed unit contemplated in the process illustrated in FIG. 2 since the PSA effluent in the subject process contains about 1-2 percent by volume of oxygen. Typically, the oxygen content of the PSA effluent in the present process is on the order of from about 0.01 to 1 percent by volume. Since the oxygen content is at such a low level, a small oxidation reactor consisting of a single bed without the need for catalyst regeneration over a period of several years is more than adequate in the method of this invention.

The effluent from the selective oxidation reactor, predominately propane and propylene, is recycled to the dehydrogenator. In the embodiment shown in Fig. 3, this recycle stream is combined with fresh feed and admitted to the dehydrogenator. In an alternative embodiment of the subject invention, the recycle stream is introduced into the latter stage of a multistage dehydrogenator as will be discussed hereafter.

The oxygen-containing stream produced by the PSA system of this invention generally contains from 60 to 95 percent of the unreacted oxygen in the quench tower effluent, depending on the amount of nitrogen present. The fact that the process of this invention provides an oxygen-containing stream is advantageous for two reasons. First, the PSA system effectively removes all but less than about one percent of the oxygen present in the quench tower effluent stream. Therefore, little is required to remove the rest as described above. Second, by returning such a high percentage of unreacted oxygen to the oxidation/ammoxidation reactor, the PSA process of this invention significantly increases the overall efficiency of the process when the feed to the reactor contains a high percentage of oxygen. These benefits are realised by all embodiments of the present invention.

In the embodiment of the present invention illustrated in Fig. 4, a second PSA system is added to the process shown in Fig. 3. This second PSA system is located between the dehydrogenator and the oxidation/ammoxidation reactor. Although the process shown in Fig. 4 is a modification of that shown in Fig. 3, a second PSA system may likewise be added to the system illustrated in Fig. 5 to function in a like manner. The second PSA system present in the process illustrated in Fig. 4 contains as adsorbent, such as silica gel or activated carbon, which will strongly adsorb $C_3$ hydrocarbons and permit hydrogen to pass through. The resulting hydrogen-enriched stream may be vented or partially recycled as described herein, i.e., to the selective oxidation reactor or the dehydrogenator. When the optional second PSA system is present in the system, the novel PSA system of the present invention will not produce a hydrogen-enriched stream.

Turning to Fig. 5, the dehydrogenator utilised is a multistage unit which eliminates the need for the selective oxidation reactor. The use of a multistage catalytic reactor is described in the literature, e.g. Pujado et al in a paper entitled "Catalytic Conversion of LPG" presented at the American Institute of Chemical Engineers, April 6-10, 1986. In such reactors, the catalyst sequentially flows through a series of discrete reactors and is withdrawn at the end for regeneration and recycle. The reactant gas stream likewise flows through the reactors and is withdrawn into a heating means between each of the individual reactors. The dehydrogenator typically operates at a temperature of from 500° to 800°C., preferably from 600° to 700°C. The reheating of the reactant stream as it flows through the reactors is especially beneficial for an endothermic reaction such as the conversion of propane to propylene.

In the multistage dehydrogenator shown in Fig. 5, the reactant gas stream does not flow through all of the reactors, but is withdrawn as a product stream intermediate the first and last reactors. Preferably, there are at least four reactors and the product stream is withdrawn from the penultimate reactor. It is beneficial to withdraw the product stream from a latter stage of the dehydrogenator to obtain maximum efficiency therefrom. The reheating of the reactor stream takes place only up to and including the reactor from which the product stream is withdrawn.

The first stream from the PSA system of the present invention, which comprises of unreacted alkane and alkene and a minor amount of oxygen, is introduced into the reactor following that from which the product stream is withdrawn, passed therethrough and through subsequent reactors, if any. The low oxygen content thereof can be eliminated without detriment to the system. Therefore, the selective oxidator present in the embodiments of the present invention shown in Figs. 3 and 4 can be eliminated. The hydrogen-enriched stream produced in the PSA system may be introduced into the dehydrogenator, taken as product, or vented. The detail of the multistage dehydrogenator utilised in accordance with the present invention is shown in Fig. 6.

In the embodiment shown in Fig. 6, the effluent from the final reactor of the dehydrogenator is introduced into the initial feed stream. In the event that the feed to one of the intermediate reactors more closely approximates the effluent in regard to the concentration of the alkene than the initial feed, the effluent is introduced into such intermediate reactor. It is further contemplated to introduce the effluent from the final reactor directly into the ammoxidation/oxidation reactor if the alkene content thereof is sufficiently high. This might occur, for example, when the PSA effluent passes through two or more reactors of the dehydrogenator.

The hydrocarbon recycle stream from the PSA system of this invention contains practically no hydrogen. Therefore, a portion of the hydrogen stream produced by the PSA system may be combined with the hydrocarbon recycle stream and re-introduced into the multistage dehydrogenerator. A portion of the hydrogen stream can likewise be introduced into the initial feed to the multistage dehydrogenator to prolong the life of the catalyst therein.

It is contemplated herein, although not necessary, to add a second PSA unit on the effluent from the dehydrogenator to remove hydrogen therefrom. The hydrogen thus obtained may be vented, recycled to the dehydrogenator feed or supplied to the heater in combination with an oxygen feed for combustion. As in the embodiment shown in FIG. 4, the first PSA system will not produce a hydrogen-enriched stream when the second PSA unit is present. It will be appreciated by those skilled in the art that a single heater can be utilised in FIG. 6 with all streams flowing therethrough.

Utilising a system as shown in FIG. 3 for the production of acrylonitrile utilising propane as the starting material, the flow rates at various points in the system were determined and are presented in Table I. The flow rates are expressed in mole percent based on 100 moles of acrylonitrile produced. The propane feed was virtually 100 percent propane. The fresh feed added to the dehydrogenator effluent prior to introduction into the ammoxidation reactor was 32.88 percent of ammonia and 67.12 percent of pure oxygen. The data expressed in Table I represents operation of the system under conditions such that 80 percent and 97 percent, respectively, of the propylene in the feed to the ammoxidation reactor is converted to different products, including acrylonitrile, in the ammoxidation reactor.

In Table I, Point A is the feed into the dehydrogenator after the stream from the selective oxidation reactor has been combined with fresh propane, Point B is the combined feed into the ammoxidation reactor, Point C is the ammoxidation reactor effluent, Point D is the quench tower gaseous effluent to the PSA system, Point E is the hydrocarbon-rich recycle stream from the PSA system and Point F is the oxygen-enriched recycle from the PSA system. As previously mentioned, the amount of hydrogen in the feed to the dehydrogenator will vary with the catalyst and reaction conditions used, and may be negligible. For purposes of the comparative results given in Tables I, II and III, the hydrogen to propane ratio in the dehydrogenator feed, Point A was kept at about 0.5.

TABLE I

80 Percent Conversion Pure Oxygen Feed

| Component | A | B | C | D | E | F |
|---|---|---|---|---|---|---|
| Propylene | 0.7 | 10.0 | 0.3 | 0.5 | 1.7 | -- |
| Propane | 61.6 | 14.1 | 13.4 | 21.5 | 79.2 | 0.9 |
| Oxygen | --- | 20.2 | 4.0 | 8.0 | 3.2 | 9.3 |
| CO | 0.2 | 0.4 | 1.1 | 1.2 | 0.5 | 1.4 |
| $CO_2$ | 4.0 | 1.7 | 3.7 | 3.3 | 9.2 | 0.7 |
| Acrylonitrile | --- | --- | 6.5 | --- | --- | --- |
| Acrolein | --- | -- | 0.1 | --- | --- | --- |
| Acetonitrile | --- | -- | 0.1 | --- | --- | --- |
| HCN | --- | -- | 1.5 | --- | --- | --- |
| Water | 2.8 | 1.1 | 27.4 | --- | --- | --- |
| Ammonia | --- | 9.5 | 1.0 | --- | --- | --- |
| Methane | 0.2 | 0.8 | 0.8 | 1.3 | 0.5 | 1.5 |
| Ethane | 0.3 | 1.0 | 1.0 | 1.6 | 0.6 | 1.8 |
| Ethylene | 0.1 | 0.2 | 0.2 | 0.3 | 0.1 | 0.4 |
| Hydrogen | 30.2 | 40.9 | 39.0 | 62.4 | 5.0 | 84.1 |
| TOTAL | 589.9 | 1545.0 | 1623.4 | 1013.3 | 253.4 | 384.8 |

97 Percent Conversion Pure Oxygen Feed

| Component | A | B | C | D | E | F |
|---|---|---|---|---|---|---|
| Propylene | 0.6 | 8.7 | 0.2 | 0.4 | 1.4 | --- |
| Propane | 60.2 | 14.0 | 13.1 | 21.1 | 75.1 | 0.9 |
| Oxygen | --- | 21.4 | 4.0 | 8.1 | 3.1 | 9.5 |
| CO | 0.2 | 0.4 | 1.2 | 1.2 | 0.5 | 1.4 |
| $CO_2$ | 6.2 | 2.7 | 5.7 | 5.2 | 14.1 | 1.1 |
| Acrylonitrile | --- | --- | 6.0 | --- | --- | --- |
| Acrolein | --- | -- | 0.1 | --- | --- | --- |
| Acetonitrile | --- | -- | 0.1 | --- | --- | --- |
| HCN | --- | -- | 1.3 | --- | --- | --- |
| Water | 2.8 | 1.1 | 27.5 | --- | --- | --- |
| Ammonia | --- | 9.0 | 1.0 | --- | --- | --- |
| Methane | 0.2 | 0.8 | 0.8 | 1.2 | 0.5 | 1.4 |
| Ethane | 0.3 | 1.0 | 1.0 | 1.5 | 0.6 | 1.8 |
| Ethylene | 0.1 | 0.2 | 0.2 | 0.3 | 0.1 | 0.4 |
| Hydrogen | 29.5 | 40.7 | 37.9 | 61.0 | 4.7 | 83.4 |
| TOTAL | 606.0 | 1559.3 | 1672.2 | 1039.1 | 268.4 | 388.8 |

Again utilising a system as shown in FIG. 3 for the production of acrylonitrile with propane as a starting material, the oxygen feed to the ammoxidation reactor was changed to an equal mixture of pure oxygen and air which produced oxygen-enriched air containing approximately 60 percent by volume of oxygen. The flow rates at various points in the system were determined and are presented in Table II. The data expressed in Table II represents operation of the system under conditions such that 80 percent and 97 percent, respectively, of the propylene in the feed to the ammoxidation reactor is converted therein to different products, including acrylonitrile.

8

TABLE II

80 Percent Conversion Equal Parts Pure Oxygen and Air

| Component | A | B | C | D | E | F |
|---|---|---|---|---|---|---|
| Propylene | 0.7 | 8.9 | 0.3 | 0.4 | 1.5 | --- |
| Propane | 58.7 | 12.6 | 12.1 | 18.3 | 70.8 | 0.8 |
| Oxygen | -- | 18.5 | 4.0 | 7.5 | 3.2 | 8.7 |
| CO | 0.2 | 0.3 | 0.9 | 1.0 | 0.4 | 1.1 |
| $CO_2$ | 3.8 | 1.5 | 3.3 | 2.8 | 8.3 | 0.6 |
| Acrylonitrile | --- | --- | 5.9 | --- | --- | --- |
| Acrolein | --- | -- | 0.1 | --- | --- | --- |
| Acetonitrile | --- | -- | 0.1 | --- | --- | --- |
| HCN | --- | -- | 1.3 | --- | --- | --- |
| Water | 2.9 | 1.0 | 24.6 | ---- | --- | --- |
| Ammonia | --- | 8.7 | 1.0 | --- | ---- | --- |
| Methane | 0.2 | 0.6 | 0.6 | 0.8 | 0.4 | 1.0 |
| Ethane | 0.2 | 0.7 | 0.7 | 1.1 | 0.4 | 1.2 |
| Ethylene | -- | 0.2 | 0.1 | 0.2 | 0.1 | 0.2 |
| Hydrogen | 28.0 | 28.1 | 26.9 | 40.6 | 3.4 | 54.6 |
| Nitrogen | 5.3 | 18.9 | 18.2 | 27.4 | 11.5 | 31.7 |
| TOTALS | 619.0 | 1725.8 | 1800.2 | 1194.5 | 283.4 | 330.1 |

97 Percent Conversion Equal Parts Pure Oxygen and Air

| Component | A | B | C | D | E | F |
|---|---|---|---|---|---|---|
| Propylene | 0.6 | 7.7 | 0.2 | 0.4 | 1.3 | --- |
| Propane | 57.3 | 12.4 | 11.7 | 17.6 | 67.0 | 0.8 |
| Oxygen | --- | 19.4 | 4.0 | 7.5 | 3.1 | 8.8 |
| CO | 0.2 | 0.3 | 1.0 | 1.0 | 0.4 | 1.2 |
| $CO_2$ | 5.9 | 2.3 | 5.1 | 4.3 | 12.5 | 1.0 |
| Acrylonitrile | --- | --- | 5.3 | --- | --- | --- |
| Acrolein | --- | -- | 0.1 | --- | --- | --- |
| Acetonitrile | --- | -- | 0.1 | --- | --- | --- |
| HCN | --- | -- | 1.2 | --- | --- | --- |
| Water | 2.9 | 1.1 | 24.4 | ---- | --- | --- |
| Ammonia | --- | 8.0 | 1.0 | --- | --- | --- |
| Methane | 0.2 | 0.6 | 0.5 | 0.8 | 0.3 | 0.9 |
| Ethane | 0.2 | 0.7 | 0.7 | 1.0 | 0.4 | 1.2 |
| Ethylene | --- | 0.1 | 0.1 | 0.1 | 0.1 | 0.2 |
| Hydrogen | 27.2 | 27.5 | 25.9 | 39.0 | 3.2 | 53.0 |
| Nitrogen | 5.5 | 19.9 | 18.8 | 28.2 | 11.7 | 33.0 |
| TOTAL | 637.5 | 1764.4 | 1872.7 | 1244.5 | 300.9 | 341.0 |

Utilising a system as shown in FIG. 4 for the production of acrylonitrile using propane as the starting material, the flow rates at various points in the system are presented in Table III. The propane, ammonia and pure oxygen feeds were as in Table I. The system was operated to convert 80 and 97 percent, respectively, of the propylene feed to the ammoxidation reactor to products.

In Table III, Point A is the feed into the dehydrogenator after the recycle stream has been combined therewith, Point B is the dehydrogenator effluent, Point C is the total feed into the ammoxidation reactor, Point D is the ammoxidation reactor effluent, Point E is the quench tower gaseous effluent, and Point F is the hydrocarbon-rich recycle stream to the selective oxidation reactor, and Point G is the oxygen-containing gas recycle from the PSA system.

## TABLE III

### 80 Percent Conversion Pure Oxygen Feed

| Component | A | B | C | D | E | F | G |
|---|---|---|---|---|---|---|---|
| Propylene | 0.7 | 18.7 | 11.2 | 0.3 | 0.5 | 1.7 | --- |
| Propane | 60.6 | 30.2 | 15.7 | 14.7 | 25.3 | 78.5 | 1.2 |
| Oxygen | 1.2 | --- | 22.3 | 4.0 | 8.6 | 2.9 | 10.8 |
| CO | 0.2 | 0.2 | 0.7 | 1.5 | 1.4 | 0.5 | 1.7 |
| $CO_2$ | 2.9 | 3.2 | 2.0 | 4.2 | 3.9 | 9.2 | 0.9 |
| Acrylonitrile | --- | --- | --- | 7.2 | --- | --- | --- |
| Acrolein | --- | --- | --- | 0.1 | --- | --- | --- |
| Acetonitrile | --- | --- | --- | 0.1 | --- | --- | --- |
| HCN | --- | --- | --- | 1.6 | --- | --- | --- |
| Water | --- | --- | --- | 30.6 | --- | --- | --- |
| Ammonia | --- | --- | 10.6 | 1.0 | --- | --- | --- |
| Methane | 0.7 | 1.4 | 2.9 | 2.7 | 4.7 | 1.6 | 5.9 |
| Ethane | 0.8 | 1.7 | 3.7 | 3.4 | 5.9 | 2.0 | 7.4 |
| Ethylene | 0.2 | 0.3 | 0.7 | 0.7 | 1.2 | 0.4 | 1.5 |
| Hydrogen | 31.7 | 44.4 | 30.1 | 28.0 | 48.4 | 3.3 | 70.6 |
| TOTAL | 599.7 | 723.2 | 1373.8 | 1474.0 | 852.9 | 253.1 | 517.2 |

### 97 Percent Conversion Pure Oxygen Feed

| Component | A | B | C | D | E | F | G |
|---|---|---|---|---|---|---|---|
| Propylene | 0.6 | 18.3 | 9.7 | 0.3 | 0.5 | 1.4 | --- |
| Propane | 59.2 | 29.6 | 15.6 | 14.2 | 24.7 | 74.4 | 1.2 |
| Oxygen | 1.2 | --- | 23.6 | 4.0 | 8.7 | 2.9 | 11.1 |
| CO | 0.2 | 0.2 | 0.8 | 1.5 | 1.4 | 0.5 | 1.8 |
| $CO_2$ | 6.1 | 5.1 | 3.2 | 6.5 | 6.1 | 14.1 | 1.5 |
| Acrylonitrile | --- | --- | --- | 6.6 | --- | --- | --- |
| Acrolein | --- | --- | --- | 0.1 | --- | --- | --- |
| Acetonitrile | --- | --- | --- | 0.1 | --- | --- | --- |
| HCN | --- | --- | --- | 1.4 | --- | --- | --- |
| Water | --- | --- | --- | 30.6 | --- | --- | --- |
| Ammonia | --- | --- | 9.9 | 1.0 | --- | --- | --- |
| Methane | 0.7 | 1.3 | 2.9 | 2.6 | 4.6 | 1.5 | 5.8 |
| Ethane | 0.8 | 1.7 | 3.6 | 3.3 | 5.7 | 1.9 | 7.3 |
| Ethylene | 0.2 | 0.3 | 0.7 | 0.7 | 1.2 | 0.4 | 1.5 |
| Hydrogen | 31.0 | 43.5 | 29.9 | 27.2 | 47.2 | 3.1 | 69.9 |
| TOTAL | 616.1 | 739.9 | 1389.4 | 1525.9 | 879.1 | 268.1 | 524.5 |

The system shown in FIG. 4 was utilised for the production of acrylonitrile with propane as the starting material and utilising an equal mixture of pure oxygen and air is shown in Table II. The results are reported in Table IV, again operating the system to produce 80 and 97 percent conversion, respectively, of the propylene in the feed to products.

For the system shown in FIG. 4, Tables V and VI detail the compositions for combination absorber-PSA and membrane-PSA separators, respectively, in the production of acrylonitrile as a propylene conversion of 80 percent using equal parts of oxygen and air as feed. In Table V, point A is the combined feed to the dehydrogenator, B is the combined feed to ammoxidation reactor, C is the ammoxidation reactor effluent, D is the quench tower effluent, E is the hydrocarbon stream from the absorber, F is the hydrogen-enriched stream, and G is the oxygen-enriched stream. In Table VI, point A is the combined feed to the dehydrogenator, B is the combined feed to the ammoxidation reactor, C is the ammoxidation reactor effluent, D is the quench tower gaseous effluent, E is the hydrogen-enriched stream from the membrane, F is the hydrocarbon-rich recycle stream, and G is the oxygen-enriched recycle stream.

The process of this invention is advantageous in that it is very efficient and is cost attractive in comparison to prior art processes. It is readily apparent from the data presented in Tables I, II, III, IV, V and VI that, in contrast to the subject process, the process illustrated in FIG. 2 continually removes propane and propylene from the system, thereby sharply reducing the efficiency thereof. It is stated in the Khoobiar et al patent that propane and propylene are removed from the purge stream before it is vented. This would require an additional sizeable capital expenditure for the refrigeration equipment required for the recovery procedure as well as an on-going cost in power to operate the recovery unit. The process of the invention has a comparatively small incidence of build-up of any of the components of the various gaseous streams formed or separated at any stage thereof. Further, the subject process can be utilised with air or an oxygen-enriched air feed, heretofore not feasible with a closed loop system. Unexpectedly, the subject process operates at particularly enhanced efficiency with an oxygen-enriched air feed.

## TABLE IV

### 80 Percent Conversion Equal Parts Pure Oxygen and Air

| Component | A | B | C | D | E | F | G |
|---|---|---|---|---|---|---|---|
| Propylene | 0.7 | 17.5 | 9.1 | 0.3 | 0.4 | 1.4 | --- |
| Propane | 55.8 | 28.2 | 12.8 | 12.1 | 18.4 | 65.1 | 0.8 |
| Oxygen | 1.4 | --- | 18.8 | 4.0 | 7.6 | 2.9 | 9.3 |
| CO | 0.2 | 0.2 | 0.5 | 1.1 | 1.0 | 0.4 | 1.2 |
| $CO_2$ | 3.6 | 3.0 | 1.6 | 3.4 | 2.9 | 7.7 | 0.7 |
| Acrylonitrile | --- | --- | --- | 5.9 | --- | --- | --- |
| Acrolein | --- | --- | --- | 0.1 | --- | --- | --- |
| Acetonitrile | --- | --- | --- | 0.1 | --- | --- | --- |
| HCN | --- | --- | --- | 1.3 | --- | --- | --- |
| Water | --- | --- | --- | 25.1 | --- | --- | --- |
| Ammonia | --- | --- | 8.8 | 1.0 | --- | --- | --- |
| Methane | 0.4 | 1.0 | 1.2 | 1.1 | 1.7 | 0.7 | 2.1 |
| Ethane | 0.4 | 1.3 | 1.5 | 1.4 | 2.1 | 0.8 | 2.6 |
| Ethylene | 0.1 | 0.3 | 0.3 | 0.3 | 0.4 | 0.2 | 0.5 |
| Hydrogen | 28.4 | 40.9 | 9.9 | 9.3 | 14.2 | 1.1 | 20.2 |
| Nitrogen | 9.2 | 7.8 | 35.6 | 33.6 | 51.3 | 19.7 | 62.6 |
| | | | | | | | |
| TOTAL | 651.5 | 773.4 | 1692.3 | 1789.9 | 1173.9 | 304.9 | 592.4 |

97 Percent Conversion Equal Parts Pure Oxygen and Air

| Component | A | B | C | D | E | F | G |
|---|---|---|---|---|---|---|---|
| Propylene | 0.6 | 17.0 | 7.7 | 0.2 | 0.3 | 1.2 | --- |
| Propane | 54.2 | 27.6 | 12.4 | 11.5 | 17.4 | 61.4 | 0.8 |
| Oxygen | 1.4 | --- | 19.5 | 4.0 | 7.6 | 2.9 | 9.3 |
| CO | 0.2 | 0.2 | 0.5 | 1.1 | 1.0 | 0.4 | 1.2 |
| $CO_2$ | 5.6 | 4.7 | 2.5 | 5.2 | 4.3 | 11.6 | 1.0 |
| Acrylonitrile | --- | --- | --- | 5.3 | --- | --- | --- |
| Acrolein | --- | --- | --- | 0.1 | --- | --- | --- |
| Acetonitrile | --- | --- | --- | 0.1 | --- | --- | --- |
| HCN | --- | --- | --- | 1.1 | --- | --- | --- |
| Water | --- | --- | --- | 24.6 | --- | --- | --- |
| Ammonia | --- | --- | 8.1 | 1.0 | --- | --- | --- |
| Methane | 0.3 | 1.0 | 1.1 | 1.1 | 1.6 | 0.6 | 2.0 |
| Ethane | 0.4 | 1.2 | 1.4 | 1.3 | 2.0 | 0.8 | 2.5 |
| Ethylene | 0.1 | 0.3 | 0.3 | 0.3 | 0.4 | 0.2 | 0.5 |
| Hydrogen | 27.6 | 39.9 | 9.6 | 8.9 | 13.4 | 1.0 | 19.1 |
| Nitrogen | 9.7 | 8.2 | 37.0 | 34.4 | 52.0 | 20.0 | 63.7 |
| TOTAL | 672.9 | 794.8 | 1755.9 | 1889.1 | 1248.8 | 324.9 | 628.1 |

TABLE V

| PROPANE DEHYDROGENATION PROCESS/ABSORBER-PSA PROCESS PROPYLENE CONVERSION = 97% and PROPANE CONVERSION = 40% - Equal Parts Pure Oxygen and Air | | | | | | | |
|---|---|---|---|---|---|---|---|
| Component | A | B | C | D | E | F | G |
| C3H6 | 1.0 | 11.7 | 0.3 | 0.6 | 1.6 | 0.0 | 0.1 |
| C3H8 | 91.1 | 18.3 | 16.9 | 30.0 | 85.1 | 0.0 | 2.4 |
| NH3 | | 11.7 | 1.0 | 1.8 | 0.0 | 0.0 | 0.0 |
| O2 | | 26.3 | 3.0 | 5.3 | 0.6 | 2.6 | 10.4 |
| AN | | | 8.0 | 0.0 | | 0.0 | 0.0 |
| HCN | | | 1.7 | 0.0 | | 0.0 | 0.0 |
| ACR | | | 0.1 | 0.0 | | 0.0 | 0.0 |
| ACN | | | 0.1 | 0.0 | | 0.0 | 0.0 |
| CO | 0.1 | 0.0 | 1.0 | 1.8 | 0.1 | 0.9 | 3.6 |
| CO2 | 3.0 | 1.0 | 5.2 | 9.3 | 4.8 | 1.0 | 8.2 |
| H2O | | | 33.8 | 0.0 | | 0.0 | 0.0 |
| CH4 | 2.5 | 2.6 | 2.5 | 4.4 | 3.9 | 0.0 | 7.6 |
| C2H4 | 0.2 | 0.3 | 0.2 | 0.4 | 0.4 | 0.0 | 0.8 |
| C2H6 | 0.2 | 0.3 | 0.2 | 0.4 | 0.4 | 0.0 | 0.8 |
| H2 | 0.4 | 10.3 | 9.5 | 16.9 | 0.6 | 81.4 | 9.1 |
| N2 | 1.4 | 17.6 | 16.3 | 29.0 | 2.3 | 14.2 | 57.1 |
| TOTAL | 387.2 | 1159.5 | 1252.3 | 705.0 | 241.2 | 112.3 | 250.7 |

TABLE VI

| PROPANE DEHYDROGENATION PROCESS/MEMBRANE-PSA PROCESS PROPYLENE CONVERSION = 97% and PROPANE CONVERSION = 40% - Equal Parts Pure Oxygen and Air | | | | | | | |
|---|---|---|---|---|---|---|---|
| Component | A | B | C | D | E | F | G |
| C3H6 | 0.8 | 11.4 | 0.3 | 0.6 | 0.1 | 1.4 | 0.1 |
| C3H8 | 85.4 | 17.8 | 16.3 | 28.9 | 7.6 | 74.4 | 5.8 |
| NH3 | | 11.4 | 1.0 | 1.8 | 0.0 | | 0.0 |
| O2 | | 25.7 | 3.0 | 5.3 | 6.7 | 1.1 | 8.9 |
| AN | | | 7.7 | 0.0 | | | 0.0 |
| HCN | | | 1.7 | 0.0 | | | 0.0 |
| ACR | | | 0.1 | 0.0 | | | 0.0 |
| ACN | | | 0.1 | 0.0 | | | 0.0 |
| CO | 0.3 | 0.1 | 1.1 | 1.9 | 0.7 | 0.5 | 4.3 |
| CO2 | 7.1 | 2.5 | 6.4 | 11.3 | 25.9 | 11.9 | 1.5 |
| H2O | | | 34.0 | 0.0 | | | 0.0 |
| CH4 | 1.1 | 2.2 | 2.0 | 3.5 | 1.3 | 1.9 | 7.9 |
| C2H4 | 0.1 | 0.2 | 0.2 | 0.4 | 0.1 | 0.2 | 0.8 |
| C2H6 | 0.1 | 0.2 | 0.2 | 0.4 | 0.1 | 0.2 | 0.8 |
| H2 | 0.2 | 10.0 | 9.1 | 16.2 | 46.6 | 0.4 | 2.4 |
| N2 | 4.8 | 18.5 | 16.9 | 29.9 | 10.9 | 8.0 | 67.6 |
| TOTAL | 414.4 | 1190.0 | 1301.5 | 735.5 | 193.1 | 247.1 | 238.2 |

In Tables V and VI:

AN = acrylonitrile
ACR = acrolein
ACN = acetonitrile

## Claims

1. A process for the production of a nitrile or an oxide comprising the steps of:
   a) catalytically dehydrogenating gaseous alkane so as to form a gas mixture comprising alkene and unreacted alkane;
   b) reacting in the presence of a suitable catalyst the alkene content of the gas mixture with oxygen and, if a nitrile is to be produced, ammonia so as to form a gaseous reaction effluent containing said oxide or said nitrile, the source of the oxygen being pure oxygen, air or a gas enriched in oxygen relative to air;
   c) quenching said effluent in a liquid to form a liquid phase containing said nitrile or oxide and a gaseous phase;
   d) recovering said nitrile or oxide from said liquid phase;
   e) separating the gaseous phase into a first stream comprising said alkane, said alkene, and, as an impurity, oxygen; a second stream comprising 'waste' gas; and a third stream comprising oxygen;
   f) either (i) introducing the first stream into a catalytic selective oxidation unit to remove the remaining oxygen in said stream; and recycling the effluent from the selective oxidation unit to the dehydrogenator, or (ii) passing the first stream directly to the dehydrogenator.

2. A process according to Claim 1, wherein the a fourth stream comprising hydrogen is formed by the separation of the gaseous phase.

3. A process according to Claim 2, wherein the separation is performed by a pressure swing adsorption (PSA) method employing at least one bed of first adsorbent in series with at least one bed of second adsorbent, wherein the first and second streams are formed by desorption from the first adsorbent and the non-adsorbed gas is separated in said at least one bed of second adsorbent to form the third and fourth streams.

**4.** A process according to Claim 2, wherein the gaseous phase is separated by a combination of membrane and PSA methods, the membrane method being used to form the fourth stream and a feed gas stream for the PSA method, and the PSA method being used to form the first, second and third streams.

**5.** A process according to Claim 2, wherein the gaseous phase is separated by a combination of PSA and membrane methods, the PSA method includes separating the gaseous phase into the first stream, the second stream and a feed gas for the membrane method, and the membrane method producing the third and fourth streams.

**6.** A process according to Claim 2, wherein the gaseous phase is separated by a combination of absorption and PSA methods, the absorption method producing the first stream and a feed gas stream for the PSA method, and the PSA method producing the second, third and fourth streams.

**7.** A process according to Claim 2, wherein the gaseous phase is separated by a combination of cryogenic distillation and PSA methods, the cryogenic distillation method producing the first gas stream and a feed gas stream for the PSA method, and the PSA method producing the second, third and fourth gas streams.

**8.** A process according any one of Claims 2 to 7, in which the separation includes a preliminary absorption step to produce a gas stream comprising carbon dioxide and a feed gas stream which is then separated into the said first, second, third and fourth streams.

**9.** A process according to any one of the preceding claims, wherein the oxygen in the said gaseous stream is supplied from a source of pure oxygen or oxygen-enriched air, and at least a part of the third stream is recycled to the reactor used to produce the nitrile or oxide.

**10.** A process according to Claim 2, in which at least a portion of the fourth stream is passed through the selective oxidation unit with the first stream and recycled to the dehydrogenator.

**11.** A process according to any one of Claims 1 to 9, wherein the catalytic dehydrogenator comprises a series of at least three discrete catalytic reactors, a hydrocarbon stream containing said alkene and unreacted alkane is withdrawn from a reactor intermediate the first and last of said reactors and is passed to step (b), the gaseous flow between said reactors, including the reactor from which the hydrocarbon stream is withdrawn, is passed through a heating means to raise the temperature thereof, the catalyst in the dehydrogenator is passed through all of said dehydrogenator reactors, is regenerated and is recycled to the first reactor, and the reactor or reactors downstream of the one from which the said hydrocarbon stream is withdrawn receive the first said stream.

**12.** A process according to Claim 11, wherein the dehydrogenator contains at least four reactors and the hydrocarbon stream is withdrawn from the penultimate one.

**13.** A process according to Claim 11 or Claim 12, in which the gas leaving the last reactor of the catalytic dehydrogenator is recycled to the first one, or a reactor other than the first one in which the concentration of the alkene is the same as it is in the said hydrocarbon stream.

**14.** A process according to any one of Claims 1 to 9, in which the catalytic reactor comprises a series of at least three discrete catalytic dehydrogenator and a hydrocarbon stream containing said alkene and unreacted alkane is withdrawn from the most downstream reactor and is passed to step (b).

**15.** A process according to Claim 14, wherein the hydrocarbon stream has hydrogen separated from it upstream of the reaction to form the oxide or nitrile.

**16.** A process according to any one of the preceding claims, in which the third stream additionally includes methane, $C_2$ hydrocarbons and at least one oxide of carbon.

**17.** A process according to any one of the preceding claims, in which the third stream additionally includes nitrogen.

14

EP 0 372 972 B1

**18.** A process according to any one of the preceding claims in which the gaseous phase is subjected to separation at a superatmospheric pressure.

**19.** A process according to any one of the preceding claims in which the product is acrylonitrile, ethylene oxide or propylene oxide.

**Patentansprüche**

**1.** Ein Verfahren für die Produktion eines Nitrils oder eines Oxids, welches die Schritte umfaßt:

a) daß katalytisch gasförmiges Alkan dehydriert wird, um eine Gasmischung zu bilden, die Alken und nicht-reagiertes Alkan umfaßt,

b) daß in der Gegenwart eines geeigneten Katalysators der Alkengehalt der Gasmischung mit Sauerstoff und, falls ein Nitril zu produzieren ist, Ammoniak zur Reaktion gebracht wird, um einen gasförmigen Reaktionsausfluß zu bilden, der das Oxid oder das Nitril enthält, wobei die Quelle des Sauerstoffs reiner Sauerstoff, Luft oder ein Gas ist, das in Sauerstoff relativ zur Luft angereichert ist,

c) daß der Ausfluß in einer Flüssigkeit abgeschreckt wird, um eine flüssige Phase, die das Nitril oder Oxid enthält, und eine gasförmige Phase zu bilden,

d) daß das Nitril oder Oxid von der flüssigen Phase zurückgewonnen wird,

e) daß die gasförmige Phase getrennt wird in einen ersten Strom, der das Alkan, das Alken und, als eine Verunreinigung, Sauerstoff enthält, einen zweiten Strom, der 'Abfall'-Gas umfaßt, und einen dritten Strom, der Sauerstoff umfaßt,

f) daß entweder (i) der erste Strom in eine katalytische selektive Oxidationseinheit eingeführt wird, um den verbleibenden Sauerstoff im Strom zu entfernen, und der Ausfluß von der selektiven Oxidationseinheit zurückgeführt wird zur Dehydriereinrichtung, oder (ii) der erste Strom direkt zur Dehydriereinrichtung geführt wird.

**2.** Ein Verfahren nach Anspruch 1, worin ein vierter Strom, der Wasserstoff umfaßt, durch die Separation der gasförmigen Phase gebildet wird.

**3.** Ein Verfahren nach Anspruch 2, worin die Separation durch eine Druck-Schwung-Adsorptions-(PSA-)Methode durchgeführt wird, die wenigstens ein Bett eines ersten Adsorbers einsetzt in Serie mit wenigstens einem Bett eines zweiten Adsorbers, worin die ersten und zweiten Ströme durch Desorption vom ersten Adsorber gebildet werden und das nicht-adsorbierte Gas im wenigstens einen Bett eines zweiten Adsorbers getrennt wird, um die dritten und vierten Ströme zu bilden.

**4.** Ein Verfahren nach Anspruch 2, worin die gasförmige Phase durch eine Kombination von Membran- und PSA-Methoden getrennt wird, wobei die Membranmethode verwendet wird, um den vierten Strom und einen Zufuhrgasstrom für die PSA-Methode zu bilden, und die PSA-Methode verwendet wird, um die ersten, zweiten und dritten Ströme zu bilden.

**5.** Ein Verfahren nach Anspruch 2, worin die gasförmige Phase durch eine Kombination von PSA- und Membran-Methoden getrennt wird, wobei die PSA-Methode einschließt, daß die gasförmige Phase in den ersten Strom, den zweiten Strom und ein Zufuhrgas für die Membranmethode getrennt wird und die Membranmethode die dritten und vierten Ströme produziert.

**6.** Ein Verfahren nach Anspruch 2, worin die gasförmige Phase durch eine Kombination aus Absorptions- und PSA-Methoden getrennt wird, wobei die Absorptions-Methode den ersten Strom und einen Zufuhrgasstrom für die PSA-Methode und die PSA-Methode die zweiten, dritten und vierten Ströme produziert.

**7.** Ein Verfahren nach Anspruch 2, worin die gasförmige Phase durch eine Kombination von kryogenen Destillations- und PSA-Methoden getrennt wird, wobei die kryogene Destillationsmethode den ersten Gasstrom und einen Zufuhrgasstrom für die PSA-Methode und die PSA-Methode die zweiten, dritten und vierten Gasströme produziert.

**8.** Ein Verfahren nach einem der Ansprüche 2 bis 7, in welchem die Separation einen vorläufigen Absorptionsschritt einschließt, um einen Gasstrom, der Kohlendioxid umfaßt, und einen Zufuhrgasstrom zu produzieren, welcher dann in die ersten, zweiten, dritten und vierten Ströme getrennt wird.

15

**9.** Ein Verfahren nach einem der vorhergehenden Ansprüche, worin der Sauerstoff im gasförmigen Strom von einer Quelle reinen Sauerstoffs oder sauerstoffangereicherter Luft zugeführt wird, und wenigstens ein Teil des dritten Stroms zum Reaktor zurückgeführt wird, der benutzt wird, um das Nitril oder Oxid zu produzieren.

**10.** Ein Verfahren nach Anspruch 2, in welchem wenigstens ein Teil des vierten Stroms durch die selektive Oxidationseinheit hindurch mit dem ersten Strom geführt und zur Dehydriereinrichtung zurückgeführt wird.

**11.** Ein Verfahren nach einem der Ansprüche 1 bis 9, worin die katalytische Dehydriereinrichtung eine Serie von wenigstens drei getrennten katalytischen Reaktoren umfaßt, ein Kohlenwasserstoffstrom, der das Alken und nicht-reagierte Alkan enthält, einem Reaktor zwischen den ersten und letzten der Reaktoren entnommen und zu Schritt (b) geführt wird, die gasförmige Strömung zwischen den Reaktoren, einschließlich des Reaktors, dem der Kohlenwasserstoffstrom entnommen wird, durch ein Erwärmungsmittel geführt wird, um ihre Temperatur zu erhöhen, der Katalysator in der Dehydriereinrichtung durch alle der Dehydriereinrichtungs-Reaktoren hindurch geführt, regeneriert und zum ersten Reaktor zurückgeführt wird, und der Reaktor oder die Reaktoren stromabwärts des einen Reaktors, dem der Kohlenwasserstoffstrom entnommen wird, den ersten Strom aufnehmen.

**12.** Ein Verfahren nach Anspruch 11, worin die Dehydriereinrichtung wenigstens vier Reaktoren enthält und der Kohlenwasserstoffstrom dem vorletzten Reaktor entnommen wird.

**13.** Ein Verfahren nach Anspruch 11 oder Anspruch 12, in welchem das Gas, welches den letzten Reaktor der katalytischen Dehydriereinrichtung verläßt, zum ersten Reaktor zurückgeführt wird oder zu einem anderen als dem ersten Reaktor, in welchem die Konzentration des Alkens die gleiche ist wie im Kohlenwasserstoffstrom.

**14.** Ein Verfahren nach einem der Ansprüche 1 bis 9, in welchem der katalytische Reaktor eine Serie von wenigstens drei getrennten katalytischen Dehydriereinrichtungen umfaßt und ein Kohlenwasserstoffstrom, der das Alken und nicht-reagiertes Alkan enthält, dem am weitesten stromabwärts gelegenen Reaktor entnommen und zu Schritt (b) geführt wird.

**15.** Ein Verfahren nach Anspruch 14, worin der Kohlenwasserstoffstrom Wasserstoff aufweist, der von ihm stromaufwärts der Reaktion getrennt wird, um das Oxid oder Nitril zu bilden.

**16.** Ein Verfahren nach einem der vorhergehenden Ansprüche, in welchem der dritte Strom zusätzlich Methan, $C_2$-Kohlenwasserstoffe und wenigstens ein Oxid von Kohlenstoff einschließt.

**17.** Ein Verfahren nach einem der vorhergehenden Ansprüche, in welchem der dritte Strom zusätzlich Stickstoff einschließt.

**18.** Ein Verfahren nach einem der vorhergehenden Ansprüche, in welchem die gasförmige Phase einer Separation bei einem über-atmosphärischen Druck ausgesetzt wird.

**19.** Ein Verfahren nach einem der vorhergehenden Ansprüche, in welchem das Produkt Acrylnitril, Äthylenoxid oder Propylenoxid ist.

**Revendications**

**1.** Procédé de production d'un nitrile ou d'un oxyde, ce procédé comprenant les étapes consistant à :
(a) déshydrogéner catalytiquement de l'alcane gazeux de façon à former un mélange gazeux comprenant de l'alcène et de l'alcane n'ayant pas réagi ;
(b) faire réagir, en présence d'un catalyseur convenable, l'alcène contenu dans le mélange gazeux, avec l'oxygène et, si l'on doit produire un nitrile, de l'ammoniac, de façon à former un effluent gazeux de réaction contenant ledit oxyde ou ledit nitrile, la source de l'oxygène étant de l'oxygène pur, de l'air ou un gaz enrichi en oxygène par rapport à l'air ;
(c) tremper ledit effluent dans un liquide pour former une phase liquide contenant ledit nitrile ou ledit oxyde, et une phase gazeuse ;

16

EP 0 372 972 B1

(d) récupérer ledit nitrile ou ledit oxyde à partir de cette phase liquide ;

(e) séparer la phase gazeuse en un premier courant comprenant ledit alcane, ledit alcène et, à titre d'impureté , de l'oxygène ; un second courant comprenant du gaz "résiduaire" ; et un troisième courant comprenant de l'oxygène ;

(f) soit (i) introduire le premier courant dans une unité d'oxydation sélective catalytique pour enlever l'oxygène demeurant dans ce courant ; et recycler l'effluent de l'unité d'oxydation sélective vers le déshydrogénateur, soit (ii) faire passer le premier courant directement vers le déshydrogénateur.

**2.** Procédé selon la revendication 1, dans lequel un quatrième courant, comprenant de l'hydrogène, est formé par la séparation de la phase gazeuse.

**3.** Procédé selon la revendication 2, dans lequel la séparation est effectuée par une méthode d'adsorption avec variation cyclique de la pression utilisant au moins un lit d'un premier adsorbant en série avec au moins un lit d'un second adsorbant, procédé dans lequel les premier et second courants sont formés par désorption du premier adsorbant et le gaz non adsorbé est séparé dans ledit au moins un lit de second adsorbant pour former les troisième et quatrième courants.

**4.** Procédé selon la revendication 2, dans lequel la phase gazeuse est soumise à séparation par combinaison de méthodes utilisant une membrane et utilisant une adsorption avec variation cyclique de la pression, la méthode utilisant une membrane servant à former le quatrième courant et un courant de gaz d'alimentation pour la méthode d'adsorption avec variation cyclique de la pression, et la méthode d'adsorption avec variation cyclique de la pression servant à former les premier, second et troisième courants.

**5.** Procédé selon la revendication 2, dans lequel la phase gazeuse est soumise à séparation à l'aide d'une combinaison de méthode d'adsorption avec variation cyclique de la pression et de méthode utilisant une membrane, la méthode d'adsorption avec variation cyclique de la pression comprenant la séparation de la phase gazeuse en le premier courant, le second courant et un gaz d'alimentation pour la méthode utilisant la membrane, et la méthode utilisant une membrane produisant les troisième et quatrième courants.

**6.** Procédé selon la revendication 2, dans lequel la phase gazeuse est soumise à séparation par une combinaison de méthodes d'absorption et d'adsorption avec variation cyclique de la pression, la méthode d'absorption produisant le premier courant et un courant de gaz d'alimentation pour la méthode d'adsorption avec variation cyclique de la pression, et la méthode d'adsorption avec variation cyclique de la pression produisant les second, troisième et quatrième courants.

**7.** Procédé selon la revendication 2, dans lequel la phase gazeuse est soumise à séparation par une combinaison de méthodes de distillation cryogénique et d'adsorption avec variation cyclique de la pression, la méthode de distillation cryogénique produisant le premier courant gazeux et un courant de gaz d'alimentation pour la méthode d'adsorption avec variation cyclique de la pression, et la méthode d'adsorption avec variation cyclique de la pression produisant les second, troisième et quatrième courants gazeux.

**8.** Procédé selon l'une quelconque des revendications 2 à 7, dans lequel la séparation comprend une étape d'absorption préliminaire pour produire un courant gazeux comprenant du dioxyde de carbone et un courant de gaz d'alimentation qui est ensuite soumis à séparation en lesdits premier, second, troisième et quatrième courants.

**9.** Procédé selon l'une quelconque des revendications précédentes, dans lequel l'oxygène présent dans ledit courant gazeux est fourni à partir d'une source d'oxygène pur ou d'air enrichi en oxygène et au moins une partie du troisième courant est recyclée dans le réacteur servant à produire le nitrile ou l'oxyde.

**10.** Procédé selon la revendication 2, dans lequel au moins une partie du quatrième courant est envoyée traverser l'unité d'oxydation sélective avec le premier courant et est recyclée vers le déshydrogénateur.

17

**11.** Procédé selon l'une quelconque des revendications 1 à 9, dans lequel le déshydrogénateur catalytique comprend une série d'au moins trois réacteurs catalytiques séparés ("discrets"), un courant hydrocarboné contenant ledit alcène et de l'alcane n'ayant pas réagi est soutiré d'un réacteur situé entre le premier et le dernier desdits réacteurs et est envoyé vers l'étape (b), le courant gazeux circulant entre lesdits réacteurs, y compris le réacteur à partir duquel le courant hydrocarboné est soutiré, étant envoyé traverser un moyen de chauffage pour en élever la température; le catalyseur, présent dans le déshydrogénateur est envoyé traverser la totalité desdits réacteurs de déshydrogénation, est régénéré et est recyclé vers le premier réacteur; et le réacteur ou les réacteurs en aval de celui d'où ledit courant hydrocarboné est soutiré, reçoit ou reçoivent ledit premier courant.

**12.** Procédé selon la revendication 11, dans lequel le déshydrogénateur contient au moins quatre réacteurs et le courant hydrocarboné est soutiré de l'avant-dernier (pénultième).

**13.** Procédé selon la revendication 11 ou la revendication 12, dans lequel le gaz sortant du dernier réacteur du déshydrogénateur catalytique est recyclé vers le premier réacteur, ou vers un réacteur autre que le premier dans lequel la concentration de l'alcène est la même que dans ledit courant hydrocarboné.

**14.** Procédé selon l'une quelconque des revendications 1 à 9, dans lequel le réacteur catalytique comprend une série d'au moins trois déshydrogénateurs catalytiques séparés, et un courant hydrocarboné contenant ledit alcène et de l'alcane n'ayant pas réagi, est soutiré du réacteur le plus en aval et est envoyé vers l'étape (b).

**15.** Procédé selon la revendication 14, dans lequel le courant hydrocarboné subit une séparation d'hydrogène en aval de la réaction destinée à former l'oxyde ou le nitrile.

**16.** Procédé selon l'une quelconque des revendications précédentes, dans lequel le troisième courant comprend en outre du méthane, des hydrocarbures en $C_2$ et au moins un oxyde de carbone.

**17.** Procédé selon l'une quelconque des revendications précédentes, dans lequel le troisième courant contient en outre de l'azote.

**18.** Procédé selon l'une quelconque des revendications précédentes, dans lequel la phase gazeuse est soumise à séparation à une pression supérieure à la pression atmosphérique.

**19.** Procédé selon l'une quelconque des revendications précédentes, dans lequel le produit obtenu est de l'acrilonitrile, de l'oxyde d'éthylène ou de l'oxyde de propylène.

WATER  NATURAL  TO
GAS  VENT

AIR →
PROPYLENE → | AMMOXIDATION REACTOR | → | QUENCH TOWER | → | BOILER |
AMMONIA →

PRODUCT
_____

ACRYLONITRILE
ACETONITRILE
ACROLEIN
HYDROGEN CYANIDE
AMMONIA

FIG. I (PRIOR ART)

EP 0 372 972 B1

FIG. 2 (PRIOR ART)

EP 0 372 972 B1

FIG. 3

EP 0 372 972 B1

PROPANE

DEHYDROGENATOR

PROPANE
PROPYLENE
HYDROGEN

SELECTIVE
OXIDATION
REACTOR

HYDROGEN

PSA UNIT

AMMONIA

OXYGEN

AMMOXIDATION
REACTOR

OXYGEN

WATER

PROPANE
PROPYLENE
HYDROGEN
OXYGEN

PSA UNIT

QUENCH
TOWER

CARBON OXIDES
$C_{1-2}$ HYDROCARBONS
HYDROGEN

PRODUCT

FIG. 4

EP 0 372 972 B1

FIG. 5

FIG. 6

EP 0 372 972 B1